# EUROPÄISCHE PATENTSCHRIFT

(11) **EP 2 178 611 B1**
(45) Veröffentlichungstag und Bekanntmachung des Hinweises auf die Patenterteilung: **02.10.2019**
(21) Anmeldenummer: 08785343.8
(22) Anmeldetag: 05.08.2008
(51) Int. Cl.: B01D 3/40, C07C 7/08, C07C 15/04, C07C 15/06, C07C 15/08, C10G 7/08

(54) **GEWINNUNG VON BENZOL UND BENZOLABKÖMMLINGEN AUS BENZINFRAKTIONEN UND RAFFINERIESTRÖMEN**
EXTRACTION OF BENZOL AND BENZOL DERIVATIVES FROM GASOLINE FRACTIONS AND REFINERY FLOWS
RÉCUPÉRATION DE BENZÈNE ET DE DÉRIVÉS BENZÉNIQUES À PARTIR DE FRACTIONS D'ESSENCE ET DE COURANTS DE RAFFINERIE

(30) Priorität: 17.08.2007 DE 102007039074
(43) Veröffentlichungstag der Anmeldung: 28.04.2010
(73) Patentinhaber: thyssenkrupp Industrial Solutions AG, 45143 Essen (DE)
(72) Erfinder: NOLL, Oliver, 44579 Castrop-Rauxel (DE); GEHRKE, Helmut, 59192 Bergkamen (DE); LÜBBECKE, Christian, 59269 Beckum (DE); KOLBE, Bärbel, 58452 Witten (DE)
(74) Vertreter: thyssenkrupp Intellectual Property GmbH
(86) Internationale Anmeldenummer: PCT/EP2008/006415
(87) Internationale Veröffentlichungsnummer: WO 2009/024259

(56) Entgegenhaltungen:
- WO-A-01/83642
- DE-C1- 4 437 702
- FR-A- 1 376 123
- US-A- 2 439 534
- US-A- 2 660 581
- US-A- 2 842 484
- B.G. Kyle ET AL: "Solvent Selection for Extractive Distillation", Industrial and Engineering Chemistry, 1 February 1965 (1965-02-01), pages 43-48, XP055267763, DOI: 10.1021/ie50662a007 Retrieved from the Internet: URL:http://pubs.acs.org/doi/pdf/10.1021/ie 50662a007 [retrieved on 2016-04-22]
- F.-M Lee: "Extractive distillation" In: "Special Distillation Processes", 1 January 2000 (2000-01-01), Elsevier, XP055265646, ISBN: 978-0-444-51648-0 pages 1013-1022,

## Beschreibung

Die Gewinnung von Aromaten aus Benzinfraktionen und Raffinerieströmen stellt einen wichtigen Verfahrensschritt in der Petrochemie und in der Kokerei- und Raffinerietechnik dar. Insbesondere Benzol und einfache Abkömmlinge des Benzols sind wichtige Rohstoffe für die Herstellung von Farbstoffen, Kunststoffen, Lösungsmitteln und Lacken. Da diese Verbindungen in aromatenhaltigen Fraktionen häufig im Gemisch mit nichtaromatischen Verbindungen vorkommen, sind Verfahrensschritte zu deren Isolierung von großer Bedeutung. Beispiele für aromatenhaltige Fraktionen sind Reformat- und Pyrolysebenzin, aber auch Destillationsfraktionen aus Mineralölen oder Kokereibenzol.

Eine einfache destillative Abtrennung von Aromaten aus aromatenhaltigen Benzinfraktionen ist nicht möglich, weil das Benzin oder dessen Fraktionen aus einer Vielzahl Stoffen besteht, deren Siedebereich eng beieinander liegt. Folglich müssen zur Trennung Verfahren eingesetzt werden, die sich weitere physikalische Effekte zunutze machen. Zur technischen Ausführung stehen mehrere Verfahren bereit, die auf unterschiedlichen physikalischen Trennverfahren beruhen. Die wichtigsten sind die Azeotropdestillation, die Flüssig-/Flüssigextraktion und die Extraktivdestillation.

Bei der Azeotropdestillation wird dem zu trennenden Gemisch ein Lösungsmittel hinzugegeben, das mit der aliphatischen oder aromatischen Komponente ein Gemisch mit konstantem Siedepunkt bildet. Dieses Azeotrop wird destillativ von dem Ausgangsgemisch abgetrennt und nach der Destillation in Azeotropbildner und aromatische Fraktion gespalten. Bei einer Flüssig-/Flüssigextraktion wird das zu trennende Gemisch mit einem Lösungsmittel versehen, das ein Zweiphasengemisch ausbildet und für eine Komponente eine höhere Löslichkeit aufweist und diese somit aus dem Lösungsmittelgemisch extrahiert. Die aromatische Komponente kann nach der Extraktion beispielsweise durch eine Destillation von dem Lösungsmittel abgetrennt werden.

Die Extraktivdestillation macht sich das Phänomen zunutze, dass sich in einem Gemisch hierzu geeigneter Komponenten die Fugazitäten der einzelnen Bestandteile ändern. Die Fugazität wird dabei als der korrigierte Partialdampfdruck in der Mischung verstanden. Ursache für die Änderung der Fugazität ist die Tatsache, dass zwischen den einzelnen Molekülarten unterschiedliche abstoßende Wechselwirkungen herrschen. Eine Mischungskomponente, die bezüglich der anderen Bestandteile größere Abstoßungskräfte aufweist, geht deshalb leichter in die Dampfphase über als eine Komponente, die geringere Abstossungskräfte aufweist.

Bei einer Extraktivdestillation wird ein Lösungsmittel zugegeben, von dem man weiß, dass es die Fugazität einer oder mehrerer Komponenten selektiv zu erhöhen zu vermag. Bei aromatenhaltigen Kohlenwasserstoffgemischen weisen oft die aliphatischen Bestandteile des Gemisches höhere Abstossungskräfte gegenüber dem Lösungsmittel auf, so dass sich dadurch deren Fugazität deutlich erhöht. Die Fugazität der aromatischen Komponente hingegen verändert sich vergleichsweise weniger. Aus diesem Grund gehen die aliphatischen Bestandteile bei einer Destillation mit dem Lösungsmittel bevorzugt in das Raffinat, das leichtsiedende Kopfprodukt einer Destillation über, während sich die aromatischen Bestandteile im Extrakt, dem schwerer siedenden Sumpfprodukt einer Destillation, befinden. Voraussetzung hierfür ist der Einsatz eines Lösungsmittels, das den erwünschten Effekt aufweist, indem es die Fugazitäten der einzelnen Komponenten in der gewünschten Weise verändert.

Eine Extraktivdestillation weist gegenüber einer Azeotropdestillation oder einer Flüssig-/Flüssigextraktion häufig Vorteile auf. So ist bei einer Extraktivdestillation der Massentransfer häufig wesentlich größer als bei einer Azeotropdestillation, weil bei ersterer die angewandten Temperaturen deutlich höher sind. Für eine Extraktivdestillation ist der apparative Aufwand erheblich geringer als für eine Flüssig-/Flüssigextraktion, da man statt einer Extraktionskolonne mit jeweils nachfolgender Destillation in der Regel nur zwei Destillationskolonnen benötigt. Da bei einer Extraktivdestillation erheblich weniger Lösungsmittel als bei einer Flüssig-/Flüssigextraktion benötigt wird, liegen die Kosten für Installation und Betrieb deutlich niedriger.

Das zentrale Problem zur Durchführung einer Extraktivdestillation ist die Wahl eines geeigneten Lösungsmittels. Aus der Vielzahl der möglichen Lösungsmittel ist dasjenige herauszufinden, das die vorgesehene Trennung mit einem Minimum an zirkulierendem Lösungsmittel ermöglicht. Entscheidende Kriterien hierfür sind die Kapazität und die Selektivität eines Lösungsmittels. Die Kapazität gibt an, wie sich die aromatische Komponente in flüssigem Aggregatzustand zwischen den einzelnen Phasen gemäß dem Nernst'schen Verteilungssatz verteilt. Je höher die Kapazität ist, desto besser löst sich die aromatische Komponente im Lösungsmittel und desto weniger Lösungsmittel wird benötigt. Je niedriger die Kapazität ist, desto eher bildet sich mit den aromatischen Komponenten und dem Lösungsmittel im flüssigen Zustand ein Zweiphasengemisch. Die Kapazität bestimmt also im Wesentlichen die Menge des benötigten Lösungsmittels.

Die Selektivität gibt an, wie sich die Extraktion der gewünschten Übergangskomponente verbessert im Vergleich zu den anderen im Raffinat enthaltenen Komponenten. Je größer die Selektivität eines Extraktionsmittels ist, desto stärker ist die Abstoßung der aliphatischen Komponente und damit deren Veränderung der Fugazität. Die Selektivität bestimmt im Wesentlichen die Trennwirkung und damit die für die extraktive Destillation notwendige Anzahl an theoretischen Böden. Mit sinkender Selektivität steigt somit der apparative Aufwand.

Verschiedene Lösungsmittel, die sich für eine extraktive Destillation von Aromaten eignen, sind bekannt. Häufig angewendete Lösungsmittel sind Diethylenglykol, Dimethylsulfoxid, Sulfolan, N-Methylpyrrolidon, Dimethylacetamid und N-Formylmorpholin. Als Einsatzgemisch wird ein Kohlenwasserstoffstrom vorgelegt, der aromaten- und aliphatenhaltig ist und in einer Vordestillation in einen Kohlenwasserstoff mit einem relativ eng begrenzten Siedebereich destilliert wird. Je nach Trennwirkung der Extraktivdestillation kann ein C₆-Strom, ein (C₆-C₇)-Strom oder ein (C₆-C₈)-Strom vorgelegt werden.

Die eigentliche Apparatur für die Extraktivdestillation besteht in der Regel aus zwei Destillationskolonnen. In der ersten Kolonne wird die eigentliche Extraktivdestillation durchgeführt. Dabei erhält man am Kopf der Kolonne einen Raffinatstrom, der überwiegend aus nichtaromatischen Kohlenwasserstoffen und, je nach Ausführungsart, etwas Lösungsmittel besteht. Da die abstoßende Wirkung des Lösungsmittels für die nichtaromatischen Kohlenwasserstoffe größer ist, gehen diese Verbindungen leichter in die Dampfphase über. Am unteren Teil der Kolonne erhält man ein Gemisch, das überwiegend aus aromatischen Verbindungen und dem extrahierenden Lösungsmittel besteht. Dieses Gemisch wird dann in eine Stripperkolonne geleitet, wo das aromatenhaltige Gemisch von dem Lösungsmittel destillativ getrennt wird. Das Lösungsmittel wird wieder in die erste Kolonne zurückgeführt.

Nach der destillativen Trennung des Extrakts erhält man ein aromatenreiches Kohlenwasserstoffgemisch als Fraktion am Kopf der Stripperkolonne und eine a-romatenarme Lösungsmittelfraktion als Sumpfprodukt. Beide Fraktionen können zur weiteren Reinigung nachbehandelt werden. Die Aromaten können nach der Wäsche beispielsweise destillativ aufgearbeitet werden, so dass man die einzelnen Aromaten je nach Alkylierungsgrad und Siedepunkt erhält. Dabei erhält man die Benzolabkömmlinge Benzol, Toluol und Xylol. Zur Trennung der Xylolisomeren können sich weitere Prozessschritte anschließen. Als Reinigungsschritt für die Aromatenfraktion kann sich auch ein Waschprozess mit Wasser eignen.

Die DE 1568940 C3 beschreibt einen Prozess zur extraktiven Destillation von Aromaten mit N-Formylmorpholin als Lösungsmittel. Der Prozess kann sowohl zur Isolierung von Aromaten aus einer aromatenhaltigen Ausgangsfraktion als auch zur Reinigung von Kohlenwasserstoffströmen von Aromaten dienen. Zur Anwendung kommt dieser Prozess in einer Vorrichtung, die eine Kolonne zur extraktiven Destillation, einen Lösungsmittelabtreiber, einer Stripperkolonne und eine Lösungsmittelregenerationskolonne enthält. Die erhaltenen Aromaten können je nach Reinheit und Anforderung direkt erhalten werden oder einer Nachbehandlung unterzogen werden. Dieses Verfahren verlangt durch die relativ niedrige Lösungsmittelkapazität eine erhöhte Lösungsmittelmenge und einen erhöhten konstruktiven Aufwand.

Die EP 679708 A1 beschreibt einen Extraktionsprozess, der durch eine spezielle apparative Anordnung mit nur einer Extraktionskolonne auskommt. Die Extraktion wird in einer Kolonne durchgeführt, aus der ein aliphatenreiches Kopfprodukt und im mittleren Kolonnenbereich ein aromatenreiches Seitenprodukt erhalten wird. Das Lösungsmittel wird aus dem Sumpf über wärmetauschende Vorrichtungen in den oberen Kolonnenteil zurückgeführt. Die beiden Kohlenwasserstoffströme werden in Zyklonabscheidern und nachgeschalteten Phasenabscheidern von überschüssigem Lösungsmittel und Wasser befreit. Als Lösungsmittel werden bevorzugt Polyalkylenglykole verwendet, es können aber auch Sulfolane oder Pyrrolidone eingesetzt werden. Zur Verbesserung der Trennwirkung werden dem Lösungsmittelgemisch 0,1 bis 20 Massenprozent Wasser zugegeben. Nachteilig ist an diesem Verfahren, dass dem lösungsmittel zur Verbesserung der Trennwirkung ein gewisser Anteil an Wasser zugegeben werden muss. Dadurch sind zusätzliche Vorrichtungen zum Trocknen der erhaltenen Produkte erforderlich.

Die EP 1280869 B1 beschreibt einen Prozess zur extraktiven Destillation eines aromatenhaltigen Kohlenwasserstoffgemisches durch ein Lösungsmittelgemisch aus Sulfolan und 3-Methylsulfolan. Das Lösungsmittelgemisch kann in fast beliebigem Verhältnis eingesetzt werden und dadurch optimal an den Aromatengehalt und die Zusammensetzung des Aromatenanteils angepasst werden. Zur Anwendung kommt dieser Prozess in einer Vorrichtung, die aus einer Kolonne zur extrahierenden Destillation und einer Kolonne zur Destillation der aromatischen Fraktion dient. Durch diese Anordnung lässt sich der Prozess mit relativ geringem anlagentechnischen Aufwand führen. Nachteilig ist an diesem Verfahren ein großer Lösungsmittelumlauf und eine relativ große Kolonne zur Extraktivdestillation, da die extrahierende Lösungsmittelkombination im Verhältnis zum Kohlenwasserstoff in großem Anteil eingesetzt werden muss.

Der Erfindung liegt die Aufgabe zugrunde, ein Lösungsmittel und ein geeignetes Verfahren zur extraktiven Destillation von Benzolabkömmlingen aus Benzinfraktionen zu finden, das sowohl unter dem Gesichtspunkt der Selektivität als auch unter dem Gesichtspunkt der Kapazität verbesserte Eigenschaften besitzt. Die Kapazität des Lösemittels soll ausreichend hoch sein, so dass man mit relativ wenig Lösungsmittelumlauf auskommt. Die Kosten des Lösemittels sollen niedrig und der apparative Aufwand gering sein. Die erzielte Selektivität des Lösemittels soll hoch sein, so dass sich der Siedepunkt der aliphatischen Komponente ausreichend verschiebt und die paraffinischen und aromatischen Kohlenwasserstoffe gut aus der Benzinfraktion isolieren lassen.

Die vorliegende Erfindung löst die Aufgabe durch den Einsatz eines neuen Lösungsmittelgemisches, das die genannten Anforderungen erfüllt. Es wurde gefunden, dass die beiden Lösungsmittelzusätze N,N'-Bis-(formyl)piperazin oder 2,2'-Bis-(cyanoethyl)-ether in Kombination mit N-Formylmorpholin als Lösungsmittel für die extraktive Destillation von Aromaten besonders geeignet sind. Die Kapazität dieser Lösungsmittelkombination ist groß, so dass die Aromaten bereits mit wenig Extraktionsmittel aus der Benzinfraktion abgetrennt werden können. Die Selektivität in Bezug auf Aromaten ist hoch, so dass sich die Siedepunkte der Aromaten nach Zugabe des extrahierenden Lösungsmittels für eine destillative Trennung ausreichend unterscheiden. Durch die hohe Selektivität der erfindungsgemäßen Lösungsmittelkombination werden die nichtaromatischen Kohlenwasserstoffe fast vollständig aus der Benzinfraktion abgetrennt, so dass nach der Entfernung des extrahierenden Lösungsmittels eine einfache destillative Trennung der Aromaten Benzol, Toluol und der Xylole möglich wird.

Durch die variable selektivitätserhöhende Wirkung der erfindungsgemäßen zusätzlichen Lösungsmittelkomponenten kann der Umlauf mit Lösungsmittelgemisch soweit reduziert werden, dass man nah an der Kapazitätsgrenze oder, anders ausgedrückt, an der Entmischung von Lösungsmittel und Aromatengemisch arbeitet. Die Lösungsmittelkombination kann so optimiert werden, dass sie für jede Benzinfraktion eine maximale Einsparung an Lösungsmittelbeladung erbringt. Ein geringerer Lösungsmittelumlauf bedingt niedrigere Investitionskosten und Betriebsmittelkosten bei gleicher Anlagenkapazität und Produktreinheit.
Beansprucht wird ein Verfahren gemäß Anspruch 1.

Offenbart wird ein Verfahren zur Gewinnung einer reinen Benzol, Toluol oder Xylol oder Mischungen dieser Aromaten umfassenden Aromatenfraktion aus diese Aromaten enthaltenden Raffinerieströmen oder Benzinfraktionen durch Extraktivdestillation, wobei
- das Einsatzprodukt vor der extraktiven Destillation in einem ersten Verfahrensschritt einer Vordestillation unterworfen wird, in der die höher als die genannten Aromaten siedenden Bestandteile als Sumpfprodukt abgetrennt werden, und
- das dadurch erhaltene aromatenhaltige Ausgangsgemisch in einem zweiten Verfahrenschritt mit einem extrahierenden Lösungsmittel oder Lösungsmittelgemisch, das die Fugazität der nichtaromatischen Komponenten des Ausgangsgemisches und damit die Trennwirkung selektiv zu erhöhen vermag, vermischt und einer extraktiven Destillation unterzogen wird, und
- das extrahierende Lösungsmittel aus dem erhaltenen Extrakt in einem dritten Verfahrensschritt durch Erhöhung der Temperatur oder Erhöhung der Temperatur und Verringerung des Druckes abdestilliert wird,
dadurch gekennzeichnet, dass
die extraktive Destillation des zweiten Verfahrensschrittes mit einer Lösungsmittelkombination umfassend N,N'-Diformylpiperazin mit einem zweiten Lösungsmittel als dampfdiuckverändernder Lösungsmittelkombination durchgeführt wird, wobei das zweite Lösungsmittel zugegeben wird, um den Siedepunkt der extrahierenden Lösungsmittelkombination in einem für die Abtrennung des Lösungsmittels geeigneten Bereich zu halten, um eine Zersetzung von Lösungsmittelkomponenten zu vermeiden, wobei
die extraktive Destillation mit einem Lösungsmittelgemisch aus N,N'-Diformylpiperazin und N-Formylmorpholin durchgeführt wird.

Die erfindungsgemäßen Lösungsmittel können sowohl im Gemisch als auch allein als Lösungsmittelbestandteil eingesetzt werden. Bevorzugt werden sie im Gemisch mit einem nicht erfindungsgemäßen Lösungsmittel eingesetzt. Eine bevorzugte Lösungsmittelkombination ist N-Formylmorpholin im Gemisch mit einer der beiden erfindungsgemäßen Lösungsmittelbestandteile.

Für die neuartige Lösungsmittelkombination ergibt sich eine deutliche Einsparung an Lösungsmittelumlauf. Bei Zumischung einer Lösungsmittelkombination der Lösungsmittel N,N'-Diformylpiperazin mit N-Formylmorpholin im Massenverhältnis 1:1 ergibt sich im Vergleich zum reinen Lösungsmittel N-Formylmorpholin eine Einsparung an Lösungsmittelumlauf von 10 bis 30 Massenprozent. In einer bevorzugten Ausführung der Erfindung beträgt die Einsparung an Lösungsmittelumlauf zwischen 15 und 25 Massenprozent.

Bei Zumischung einer Lösungsmittelkombination der Lösungsmittel 2,2'-Bis-(cyanoethyl)-ether mit N-Formylmorpholin im Verhältnis 1:1 ergibt sich im Vergleich zum reinen Lösungsmittel N-Formylmorpholin eine Einsparung an Lösungsmittelumlauf von 5 bis 15 Massenprozent. In einer bevorzugten Ausführung der Erfindung beträgt die Einsparung im Vergleich zum Lösungsmittel N-Formylmorpholin zwischen 7 und 11 Massenprozent.

N,N'-Diformylpiperazin oder Hexahydro-1,4-diazin-1,4-dimethanal (HCO[cyclo-N(CH₂CH₂)₂N]CHO) ist eine leicht erhältliche Chemikalie, die häufig in der Gewinnung von Feinchemikalien eingesetzt wird. 3,3'-Oxydipropionitril oder 2,2'-Bis-(cyanoethyl)-ether (NC(C₂H₄)O(C₂H₄)CN) ist eine leicht erhältliche Chemikalie, die aufgrund ihrer speziellen polaren Eigenschaften häufig als Lösungsmittel in der Chromatographie eingesetzt wird. Die Herstellung dieser Chemikalie ist einfach, so dass bei der Bereitstellung größerer Mengen die Verbindungen preiswert erhältlich sind. Der Schmelzpunkt der bei Raumtemperatur festen Verbindung N,N'-Diformylpiperazin liegt bei 125 bis 129 °C, Schmelzpunkt und Siedepunkt der bei Raumtemperatur flüssigen Verbindung 2,2'-Bis-(cyanoethyl)-ether liegen bei -26°C und 130 bis 132°C (0,26 kPa).

Die beiden erfindungsgemäßen Lösungsmittel werden zur Ausführung des erfindungsgemäßen Verfahrens in Kombination mit einem zweiten Lösungsmittel eingesetzt, um den Siedepunkt der extrahierenden Lösungsmittelkombination in einem für die Abtrennung des Lösungsmittels geeigneten Bereich zu halten. Ist der Siedepunkt zu hoch, so kann es bei der Abtrennung der Lösungsmittel von den Aromaten zu einer Zersetzung der extrahierenden Lösungsmittel kommen. Die Zugabe eines nicht erfindungsgemäßen Lösungsmittels erniedrigt den Siedepunkt der erfindungsgemäßen Lösungsmittelkombination, so dass sich eine Zersetzung von Lösungsmittelkomponenten vermeiden lässt, ohne den Druck zu niedrig halten zu müssen.

In einer Ausführung der Erfindung werden als zweites Lösungsmittel substituierte Stickstoff- und sauerstoffhaltige Heterocyclen zugegeben. Geeignet ist als zweites Lösungsmittel insbesondere N-Formylmorpholin. Das zweite Lösungsmittel kann in einem breit variierenden Gewichtsanteil zu dem erfindungsgemäßen Lösungsmittel zugegeben werden, um den erfindungsgemäßen Effekt zu erzielen. Bevorzugt wird zur Ausführung des erfindungsgemäßen Verfahrens ein Verhältnis der beiden Lösungsmittel von 1:1.

In einer weiteren Offenbarung der Erfindung werden die erfindungsgemäßen Lösungsmittelbestandteile in Form von Derivaten eingesetzt. So ist es beispielsweise möglich, kohlenstoffhaltige Substituenten in den erfindungsgemäßen Lösungsmittelbestandteil einzubringen, ohne dass es zu einer wesentlichen Veränderung der für die Extraktion verantwortlichen Eigenschaften kommt. Um die Löslichkeit des extrahierenden Lösungsmittels in einem für das erfindungsgemäße Verfahren geeigneten Bereich zu halten, ist die Zahl der C-Atome aller Substituenten nicht größer als 7.

Offenbart wird außer dem genannten Verfahren zur Extraktivdestillation auch ein Stoffgemisch, das die Verbindungen N,N'-Diformylpiperazin und N-Formylmorpholin enthält. Gleichermaßen offenbart wird ein Stoffgemisch, das die Verbindungen N,N'-Diformylpiperazin und 2,2'-Bis-(cyanoethyl)-ether enthält. Die Vermischungen sind als Stoff in dieser Form bislang nicht bereitgestellt oder beschrieben worden. Offenbart wird außerdem die Verwendung der erfindungsgemäßen Stoffgemische für die Extraktivdestillation.

Zur Ausführung des erfindungsgemäßen Verfahrens kommt eine Vorrichtung, wie sie für extrahierende Destillationen von Aromaten typisch ist. Eine beispielhafte Vorrichtung gibt die EP 434959 A2. Das aromatenhaltige Einsatzgemisch, das aus der Vordestillation des Benzins erhalten wird, wird vorerwärmt und im unteren Teil einer für die Extraktion vorgesehenen ersten Destillationskolonne eingespeist. Diese enthält bereits die erfindungsgemäße, extrahierende Lösungsmittelkombination. Bei der extrahierenden Destillation erhält man am Kopf der Kolonne einen Raffinatstrom, der an aromatischen Kohlenwasserstoffen stark abgereichert ist und im Wesentlichen paraffinische oder auch eine gewisse Menge an naphthenischen Kohlenwasserstoffe enthält. Dieser Raffinatstrom enthält nur sehr wenig extrahierendes Lösungsmittel und kann nach Erhalt bei Bedarf einer Weiterverarbeitung, wie beispielsweise einer Wäsche, zugeführt werden. Als Sumpfprodukt der Extraktionskolonne erhält man im Gemisch mit dem extrahierenden Lösungsmittel einen an aromatischen Kohlenwasserstoffen stark angereicherten Extraktstrom. In einer Ausführung der Erfindung wird die extrahierende Destillation unter vermindertem Druck durchgeführt.

Der Extraktstrom wird in den unteren Teil einer zweiten Destillationskolonne, die auch als Stripperkolonne bezeichnet wird, eingespeist. Diese ist für die destillative Trennung des Lösungsmittels von den erwünschten Aromaten vorgesehen. Als Sumpfprodukt erhält man aus dieser Kolonne das Lösungsmittel zurück, das über eine Leitung in den oberen Teil der ersten, für die Extraktivdestillation vorgesehenen Kolonne, zurückgeführt wird. Dadurch erhält man für die extrahierende Lösungsmittelkombination einen im Wesentlichen geschlossenen Kreislauf. Die beiden Kolonnen können zur Beheizung optional einen Reboilerkreislauf besitzen.

Als Kopfprodukt der zweiten, für die Lösungsmittelabtrennung vorgesehenen Kolonne erhält man einen im Wesentlichen lösungsmittelfreien Kohlenwasserstoffstrom, der überwiegend die erwünschten aromatischen Kohlenwasserstoffe enthält. Dieser Aromatenstrom kann nach Erhalt einer Weiterverarbeitung zugeführt werden.

In einer Ausführung der Erfindung wird der aus der Lösungsmittelabtrennung erhaltene Aromatenstrom einem optionalen Waschprozess mit Wasser zugeführt, um Reste der erhaltenen Lösungsmittelkombination zu entfernen. Dem Waschprozess können sich auch weitere Verarbeitungsschritte in Phasenabscheidern anschließen. Bevorzugt wird der erhaltene lösungsmittelfreie Extrakt nach Erhalt einer destillativen Trennung unterzogen, wobei man die einzelnen Benzolabkömmlinge in einer Menge erhält, die dem Anteil der einzelnen Aromaten am Ausgangsgemisch entspricht. Ein besonderer Vorteil des erfindungsgemäßen Verfahrens ist es, dass man auch die schwerer erhältlichen Xylole bei dieser Destillation als reine Xylolfraktion erhalten kann.

In einer weiteren Ausführung der Erfindung wird die Abtrennung des extrahierenden Lösungsmittels von den Aromaten unter vermindertem Druck durchgeführt. Dies hält die thermische Belastung der extrahierenden Lösungsmittelkombination in Grenzen und reduziert den apparativen Aufwand für die Kühlung des Extraktstroms. Um eine Durchführung der Lösungsmittelabtrennung in der zweiten Destillationskolonne unter reduziertem Druck zu ermöglichen, wird der aus der ersten, für die Extraktion vorgesehenen Kolonne erhaltene Raffinatstrom mit einer geeigneten Vorrichtung auf einen niedrigeren Gasdruck zurückgeführt.

Der erfindungsgemäße Effekt konnte durch theoretische Berechnungen mit VLE-Daten (Dampf-Flüssigkeitsgleichgewicht) unterstützt werden. Zugrunde gelegt wurde dabei ein simulierter Prozess für das erfindungsgemäße Verfahren, der durch Eingabe der wichtigsten anlagentechnischen Parameter auf einem Rechenprogramm Aspen Plus der Firma Aspen Tech durchgerechnet wurde. Eingegeben wurden dabei die Parameter Temperatur, Druck, Siedepunkt, Wechselwirkungsparameter und Löslichkeit aller angegebenen Verbindungen. Zur Anwendung kam in dem simulierten Prozess eine Destillationsanlage, die typisch ist für einen Extraktionsprozess für einfache aromatische Kohlenwasserstoffe. Zur Demonstration des erfindungsgemäßen Effektes wurden das reine Lösungsmittel N-Formylmorpholin mit den erfindungsgemäßen Lösungsmittelkombinationen N,N'-Diformylpiperazin mit N-Formylmorpholin und 2,2'-Bis-(cyanoethyl)-ether mit N-Formylmorpholin verglichen. Beide Lösungsmittelkombinationen wurden für die Simulationsrechnung in einem Massenverhältnis von 1:1 eingesetzt. Als Ergebnis wurden Lösungsmittelmengen an wichtigen Prozesspunkten erhalten, die für die errechnete Ersparnis des Lösungsmittelumlaufs in Gewichtsprozent umgerechnet wurden. Die Fehlertoleranz für dieses Rechenprogramm betrug für die angeführten Prozentzahlen der Lösungsmitteleinsparung ± 10 %.

Um die energetische Bilanz des Prozesses günstig zu gestalten, kann das erhaltene heiße Sumpfprodukt aus der Lösungsmittelabtrennung, das im Wesentlichen aus extrahierendem Lösungsmittel besteht, über wärmetauschende Vorrichtungen in den Prozess zurückgeführt werden. Das heiße Sumpfprodukt aus der Stripperkolonne kann zum Aufheizen des Einsatzgemisches, zum Erhitzen der ersten, für die extraktive Destillation vorgesehenen Kolonne oder zum Erhitzen der zweiten, für die Lösungsmittelabtrennung vorgesehenen Kolonne eingesetzt werden.

Bei einem kontinuierlichen Betrieb der Anlage zur Aromatengewinnung kann es vorkommen, dass sich trotz aller Maßnahmen ein geringer Verlust an extrahierendem Lösungsmittel einstellt. So kann Lösungsmittel über die Raffinatströme aus den niedrigsiedenden Fraktionen in die für die Weiterverarbeitung vorgesehenen Prozesse gelangen. Um diesen Verlust auszugleichen, kann über eine geeignete Vorrichtung vorerwärmtes frisches Lösungsmittel am Kopf der ersten Kolonne in den Prozess eingespeist werden.

Die eingespeiste Menge wird dabei so gesteuert, dass das Gewichtsverhältnis von eingesetztem Lösungsmittel zu eingesetztem Kohlenwasserstoffgemisch im Bereich von 1:1 bis 5:1 liegt. In einer bevorzugten Ausführung der Erfindung liegt das Gewichtsverhältnis der eingesetzten Lösungsmittelkombination zu eingesetztem Kohlenwasserstoffgemisch bei 2:1 bis 3:1, wenn die erfindungsgemäßen Lösungsmittelkomponenten im Gemisch mit N-Formylmorpholin eingesetzt werden.

In einer Ausführung der Erfindung wird die Temperatur in der ersten, für die Extraktion vorgesehenen Kolonne bei der Destillation so eingestellt, dass das am Kopf der Kolonne ausgespeiste Raffinat bei Atmosphärendruck eine Temperatur von wenigstens 50 °C und das am unteren Ende der Kolonne erhaltene Sumpfprodukt eine Temperatur von höchstens 200 °C besitzt. Die Siedetemperaturen in der Kolonne können sich bei Anwendung eines veränderten Destillationsdrucks oder einer veränderten Zusammensetzung des Kohlenwasserstoffgemisches ändern.

In einer weiteren Ausführung der Erfindung wird die Temperatur in der zweiten, für die Lösungsmittelabtrennung vorgesehenen Kolonne so eingestellt, dass das am Kopf der Kolonne erhaltene ausgespeist Raffinat eine Temperatur von wenigstens 50°C und das am unteren Ende der Kolonne erhaltene Sumpfprodukt eine Temperatur von höchstens 260 °C besitzt. Die Maximaltemperatur wird dabei im Wesentlichen durch die Zersetzungstemperatur des Lösungsmittels bestimmt. Die Siedetemperaturen in der Kolonne können sich bei Anwendung eines veränderten Destillationsdrucks oder einer veränderten Zusammensetzung des Kohlenwasserstoffgemisches ändern.

In einer weiteren Ausführung wird das Lösungsmittel mit einem geringen Wasseranteil versehen, um die Selektivität der extraktiven Destillation zu erhöhen. Der Wasseranteil kann zwischen 0,1 bis 20 Massenprozent, bevorzugt aber 0,5 bis 10 Massenprozent betragen. Dies ist von den angewandten Bedingungen und den apparativen Gegebenheiten der eingesetzten Vorrichtung abhängig.

Der erfindungsgemäße Prozess kann sowohl zur Isolierung von Aromaten aus einer aromatenhaltigen Ausgangsfraktion als auch zur Reinigung von Kohlenwasserstoffströmen von Aromaten dienen. Die Entfernung von aromatischen Verbindungen aus einem überwiegend paraffinischen Gemisch kann beispielsweise in der Lebensmittelindustrie von Interesse sein. Als Ausgangsbenzine können sowohl Kohlenwasserstoffströme dienen, die aus der Mineralölverarbeitung und aus Raffinerien stammen, als auch Produkte, die in Kokereien oder kohlenwasserstoffproduzierenden Betrieben anfallen, wie beispielsweise Kokereibenzol.

Das erfindungsgemäße Verfahren benötigt für die Ausführung keine wesentlichen apparativen Änderungen im Vergleich zu herkömmlichen Verfahren zur extraktiven Destillation. Die Kapazität des eingesetzten Lösungsmittels steigt deutlich an, so dass ein insgesamt geringerer Lösungsmittelumlauf im gesamten Kreislauf erforderlich ist. Die eingesetzten, erfindungsgemäßen Lösungsmittelkomponenten erniedrigen die Umlaufmenge mit Lösungsmittel und erniedrigen so die Kosten. Der geringere Lösungsmittelumlauf bedingt niedrigere Investitionskosten bei gleicher Anlagenkapazität und gleicher Produktreinheit. Die Selektivität des Lösungsmittelgemisches steigt ebenfalls deutlich an, so dass man das Lösungsmittel optimal an den Aromatengehalt und der Aromatenverteilung des Einsatzgemisches anpassen kann.

Die Ausführung des erfindungsgemäßen Verfahrens zur Gewinnung von Benzolabkömmlingen aus Benzinfraktionen und Raffinerieströmen wird anhand eines Beispiels und einer Zeichnung genauer erläutert, wobei das erfindungsgemäße Verfahren nicht auf diese Ausführungsform beschränkt ist.

Beispiel: Die beigefügte Tabelle (Tabelle 1) zeigt die Einsparung an Lösungsmittel in dem erfindungsgemäßen Verfahren mit der erfindungsgemäßen Lösungsmittelkombination gegenüber herkömmlichem N-Formylmorpholin.

**Tabelle 1**

| Lösungsmittelkombination (Massenverhältnis) | Einsparung an Lösungsmittel gegenüber N-Formylmorpholin |
|---|---|
| N,N'-Diformylpiperazin + N-Formylmorpholin (1:1) | 10 bis 30 Massenprozent |
| 2,2'-Bis-(cyanoethyl)-ether+ N-Formylmorpholin (1:1) | 5 bis 15 Massenprozent |

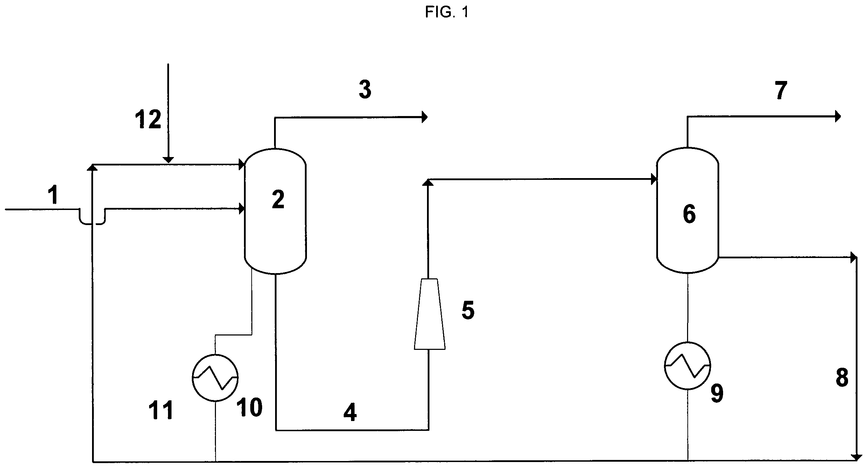

Die beigefügte Zeichnung (FIG. 1) zeigt beispielhaft eine erfindungsgemäße Ausführung des Verfahrens zur Aromatenextraktion aus einer Benzinfraktion.

Über eine Einspeiseleitung **1** wird ein aus einer Vordestillation erhaltenes aromatenhaltiges Einsatzgemisch in den mittleren Teil einer Kolonne **2** eingespeist, die für die extraktive Destillation des aromatenhaltigen Gemisches vorgesehen ist. Die Kolonne enthält bereits das erfindungsgemäße Lösungsmittel in Kombination mit einem weiteren Lösungsmittel. Bei der Destillation entsteht als Raffinat ein an Aromaten abgereichter Kohlenwasserstoffstrom **3**, der hauptsächlich paraffinische Kohlenwasserstoffe enthält und einer weiteren Verarbeitung zugeführt wird. Als Sumpfprodukt erhält man einen an Aromaten angereicherten Kohlenwasserstoffstrom **4**, der über eine Leitung mit einer entspannenden Vorrichtung **5** in einer zweite Kolonne **6** zur Lösungsmittelabtrennung geführt wird. In dieser Kolonne wird durch Erhöhung der Temperatur und/oder Erniedrigung des Druckes das Lösungsmittel abgetrennt. Man erhält als Kopfprodukt einen aromatenreichen Extrakt **7**, der weitgehend lösungsmittelfrei ist. Als Sumpfprodukt erhält man einen Lösungsmittelstrom, der im wesentlichen das an Aromaten abgereicherte Lösungsmittel erhält. Dieses wird über eine Leitung **8** in den oberen Teil der für die extraktive Destillation vorgesehenen Kolonne **2** zurückgegeben. Über wärmetauschende Einrichtungen **9, 10** und **11** kann das Lösungsmittel über einen indirekten Wärmeaustausch das eingesetzte Ausgangsgemisch **1**, die Extraktionskolonne **2** oder die Stripperkolonne **6** erhitzen. Über einen separaten Einspeisestutzen **12** kann der Lösungsmittelverlust während eines kontinuierlichen Betriebes durch Zugabe von Lösungsmittel oder Lösungsmittelbestandteilen ersetzt werden.

### Bezugszeichenliste

- 1: Einspeiseleitung für Ausgangsgemisch
- 2: Kolonne für Extraktivdestillation
- 3: Raffinat, Produktstrom mit aromatenarmem Kohlenwasserstoffgemisch
- 4: Leitung für aromatenreiches Lösungsmittelgemisch
- 5: Entspannungsvorrichtung
- 6: Stripperkolonne zur Abtrennung de Lösungsmittels
- 7: Produktstrom mit aromatenreichem Kohlenwasserstoffgemisch
- 8: Leitung für die Lösungsmittelrückführung
- 9: Wärmetauscher
- 10: Wärmetauscher
- 11: Wärmetauscher
- 12: Einspeisestutzen für Lösungsmittel

## Patentansprüche

1. Verfahren zur Gewinnung einer reinen Benzol, Toluol oder Xylol oder Mischungen dieser Aromaten umfassenden Aromatenfraktion aus diesen Aromaten enthaltenden Raffinerieströmen oder Benzinfraktionen durch Extraktivdestillation, wobei
• das Einsatzprodukt vor der extraktiven Destillation in einem ersten Verfahrensschritt einer Vordestillation unterworfen wird, in der die höher als die genannten Aromaten siedenden Bestandteile als Sumpfprodukt abgetrennt werden, und
• das dadurch erhaltene aromatenhaltige Ausgangsgemisch in einem zweiten Verfahrensschritt mit einem extrahierenden Lösungsmittel oder Lösungsmittelgemisch, das die Fugazität der nichtaromatischen Komponenten des Ausgangsgemisches und damit die Trennwirkung selektiv zu erhöhen vermag, vermischt und einer extraktiven Destillation unterzogen wird, und
• das extrahierende Lösungsmittel aus dem erhaltenen Extrakt in einem dritten Verfahrensschritt durch Erhöhung der Temperatur oder Erhöhung der Temperatur und Verringerung des Druckes abdestilliert wird,
**dadurch gekennzeichnet, dass**
• die extraktive Destillation des zweiten Verfahrensschrittes mit einer Lösungsmittelkombination umfassend N,N'-Diformylpiperazin mit einem zweiten Lösungsmittel als dampfdruckverändernder Lösungsmittelkombination durchgeführt wird, wobei das zweite Lösungsmittel zugegeben wird, um den Siedepunkt der extrahierenden Lösungsmittelkombination In einem für die Abtrennung des Lösungsmittels geeigneten Bereich zu halten, um eine Zersetzung von Lösungsmittelkomponenten zu vermeiden, wobei
• die extraktive Destillation mit einem Lösungsmittelgemisch aus N,N'-Diformylpiperazin und N-Formylmorpholin durchgeführt wird.

2. Verfahren nach Anspruch 1, **dadurch gekennzeichnet, dass** die extrahierende Destillation mit einem Massenverhältnis von Einsatzgemisch zu Lösungsmittel in einem Bereich zwischen 1:1 und 1:10 durchgeführt wird.

3. Verfahren nach einem der Ansprüche 1 bis 2, **dadurch gekennzeichnet, dass** das eingesetzte Lösungsmittel oder die eingesetzte Lösungsmittelkombination Wasser mit einem Anteil von 0,1 bis 20 Massenprozent umfasst

4. Verfahren nach einem der Ansprüche 1 bis 3, **dadurch gekennzeichnet, dass** das aus der extraktiven Destillation erhaltene aromatenreiche Gemisch nach der Lösungsmittelabtrennung einem Waschprozess mit Wasser unterzogen wird.

5. Verfahren nach einem der Ansprüche 1 bis 4, **dadurch gekennzeichnet, dass** das aus der extraktiven Destillation erhaltene aromatenreiche Gemisch nach der Lösungsmittelabtrennung in eine Benzol enthaltende erste Fraktion, eine Toluol enthaltende zweite Fraktion und eine Xylole enthaltende dritte Fraktion aufgetrennt wird.

6. Verfahren nach einem der Ansprüche 1 bis 5, **dadurch gekennzeichnet, dass** man die extraktive Destillation in einer ersten Destillationskolonne durchführt, wobei man einen an Aromaten abgereicherten Kohlenwasserstoffstrom als niedrigsiedendem Raffinat erhält.

7. Verfahren nach einem der Ansprüche 1 bis 6, **dadurch gekennzeichnet, dass** man die Lösungsmittelabtrennung in einer zweiten Destillationskolonne durchführt, wobei man einen an Aromaten angereicherten Kohlenwasserstoffstrom als niedrigsiedendem Raffinat erhält.

8. Verfahren nach einem der Ansprüche 1 bis 7, **dadurch gekennzeichnet, dass** das Lösungsmittel für die extraktive Destillation in einem geschlossenen Kreislauf geführt wird.

9. Verfahren nach einem der Ansprüche 1 bis 8, **dadurch gekennzeichnet, dass** das extrahierende Lösungsmittel aus der Lösungsmittelabtrennung als hochsiedendes Sumpfprodukt aus der zweiten Kolonne in den oberen Teil der ersten Kolonne zurückgespeist werden kann,

10. Verfahren nach einem der Ansprüche 1 bis 9, **dadurch gekennzeichnet, dass** der Lösungsmittelverlust durch Einspeisen von extrahierendem Lösungsmittel oder einem extrahierendem Lösungsmittelgemisch ersetzt werden kann.

11. Verfahren nach einem der Ansprüche 1 bis 10, **dadurch gekennzeichnet, dass** die extraktive Destillation in der ersten Kolonne bei einer Temperatur von 200 °C am unteren Ende dieser Kolonne und einer Temperatur von 50 °C am oberen Ende dieser Kolonne durchgeführt wird.

12. Verfahren nach einem der Ansprüche 1 bis 11, **dadurch gekennzeichnet, dass** die Lösungsmittelabtrennung in der zweiten Kolonne bei einer Temperatur von 260 °C am unteren Ende dieser Kolonne und einer Temperatur von 50 °C am oberen Ende dieser Kolonne durchgeführt wird.

13. Verfahren nach einem der Ansprüche 1 bis 12, **dadurch gekennzeichnet, dass** der Lösungsmittelstrom aus der Lösungsmittelabtrennung durch wärmetauschende Einrichtungen zum Erhitzen des Einsatzgemisches für die extraktive Destillation verwendet wird.

14. Verfahren nach einem der Ansprüche 1 bis 13, **dadurch gekennzeichnet, dass** der Lösungsmittelstrom aus der Lösungsmittelabtrennung durch wärmetauschende Einrichtungen zum Erhitzen der ersten Destillationskolonne für die extraktive Destillation verwendet wird.

15. Verfahren nach einem der Ansprüche 1 bis 14, **dadurch gekennzeichnet, dass** der Lösungsmittelstrom aus der Lösungsmittelabtrennung durch wärmetauschende Einrichtungen zum Erhitzen der zweiten Destillationskolonne für die Lösungsmittelabtrennung verwendet wird.

## Claims

1. Process for isolating an aromatics fraction comprising pure benzene, toluene or xylene or mixtures of these aromatics from refinery streams or petroleum fractions containing these aromatics by extractive distillation, where
• the feed is subjected to a predistillation in which the constituents boiling at a higher temperature than the specified aromatics are separated off as bottom product in a first process step before the extractive distillation and
• the resulting aromatics-containing starting mixture is mixed in a second process step with an extracting solvent or solvent mixture which is able to selectively increase the fugacity of the nonaromatic components of the starting mixture and thus the separating effect and is subjected to an extractive distillation and
• the extracting solvent is distilled off from the resulting extract in a third process step by increasing the temperature or increasing the temperature and reducing the pressure,
**characterized in that**
• the extractive distillation of the second process step is carried out using a solvent combination comprising N,N'-diformylpiperazine with a second solvent as vapour pressure-modifying solvent combination, where the second solvent is added in order to keep the boiling point of the extracting solvent combination in a range suitable for separating off the solvent in order to avoid decomposition of solvent components, where
• the extractive distillation is carried out using a solvent mixture of N, N' - diformylpiperazine and N-formylmorpholine.

2. Process according to Claim 1, **characterized in that** the extractive distillation is carried out using a mass ratio of starting mixture to solvent in the range from 1:1 to 1:10.

3. Process according to either of Claims 1 to 2, **characterized in that** the solvent used or the solvent combination used comprises water in a proportion of from 0.1 to 20% by mass.

4. Process according to any of Claims 1 to 3, **characterized in that** the aromatics-rich mixture obtained from the extractive distillation is, after the solvent removal, subjected to a scrubbing process using water.

5. Process according to any of Claims 1 to 4, **characterized in that** the aromatics-rich mixture obtained from the extractive distillation is, after the solvent removal, separated into a benzene-containing first fraction, a toluene-containing second fraction and a xylenes-third containing fraction.

6. Process according to any of Claims 1 to 5, **characterized in that** the extractive distillation is carried out in a first distillation column to give an aromatics-depleted hydrocarbon stream as low-boiling raffinate.

7. Process according to any of Claims 1 to 6, **characterized in that** the solvent removal is carried out in a second distillation column to give an aromatics-enriched hydrocarbon stream as low-boiling raffinate.

8. Process according to any of Claims 1 to 7, **characterized in that** the solvent for the extractive distillation is conveyed in a closed circuit.

9. Process according to any of Claims 1 to 8, **characterized in that** the extracting solvent from the solvent removal as high-boiling bottom product from the second column can be fed back into the upper part of the first column.

10. Process according to any of Claims 1 to 9, **characterized in that** the solvent loss can be replaced by feeding in of extracting solvent or an extracting solvent mixture.

11. Process according to any of Claims 1 to 10, **characterized in that** the extractive distillation in the first column is carried out at a temperature of 200°C at the lower end of this column and a temperature of 50°C at the upper end of this column.

12. Process according to any of Claims 1 to 11, **characterized in that** the solvent removal in the second column is carried out at a temperature of 260°C at the lower end of this column and a temperature of 50°C at the upper end of this column.

13. Process according to any of Claims 1 to 12, **characterized in that** the solvent stream from the solvent removal is used by means of heat-exchanging apparatuses for heating the starting mixture for the extractive distillation.

14. Process according to any of Claims 1 to 13, **characterized in that** the solvent stream from the solvent removal is used by means of heat-exchanging apparatuses for heating the first distillation column for the extractive distillation.

15. Process according to any of Claims 1 to 14, **characterized in that** the solvent stream from the solvent removal is used by means of heat-exchanging apparatuses for heating the second distillation column for the solvent removal.

## Revendications

1. Procédé pour la récupération d'une fraction d'aromatiques comprenant du benzène, du xylène ou du toluène pur ou des mélanges de ces aromatiques à partir de flux de raffinerie ou de fractions d'essence contenant ces aromatiques par distillation extractive,
- dans une première étape de procédé, le produit de départ étant soumis, avant la distillation extractive, à une prédistillation dans laquelle les constituants à point d'ébullition supérieur à celui des aromatiques mentionnés sont séparés comme produit de fond et
- dans une deuxième étape de procédé, le mélange de départ contenant des aromatiques ainsi obtenu étant mélangé avec un solvant ou un mélange de solvants d'extraction, qui permet d'augmenter sélectivement la fugacité des composants non aromatiques du mélange de départ et ainsi l'effet de séparation, et étant soumis à une distillation extractive et
- dans une troisième étape de procédé, le (s) solvant (s) d'extraction étant éliminé(s) par distillation de l'extrait obtenu par augmentation de la température ou par augmentation de la température et diminution de la pression, **caractérisé en ce que**
- la distillation extractive de la deuxième étape de procédé étant effectuée à l'aide d'une combinaison de solvants comprenant de la N,N'-diformylpipérazine conjointement avec un deuxième solvant comme combinaison de solvants modifiant la tension de vapeur, le deuxième solvant étant ajouté pour maintenir le point d'ébullition de la combinaison de solvants d'extraction dans une plage appropriée pour la séparation du/des solvant (s) afin d'éviter une décomposition des composants de solvant,
- la distillation extractive étant réalisée à l'aide d'un mélange de solvants constitué par de la N,N'-diformylpipérazine et de la N-formylmorpholine.

2. Procédé selon la revendication 1, **caractérisé en ce que** la distillation extractive est réalisée à l'aide d'un rapport massique de mélange de départ à solvant(s) dans une plage entre 1:1 et 1:10.

3. Procédé selon l'une quelconque des revendications 1 à 2, **caractérisé en ce que** le solvant utilisé ou la combinaison de solvants utilisée comprend de l'eau en une proportion de 0,1 à 20% en masse.

4. Procédé selon l'une quelconque des revendications 1 à 3, **caractérisé en ce que** le mélange riche en aromatiques obtenu à partir de la distillation extractive, après la séparation du/des solvant(s), est soumis à un procédé de lavage à l'eau.

5. Procédé selon l'une quelconque des revendications 1 à 4, **caractérisé en ce que** le mélange riche en aromatiques obtenu à partir de la distillation extractive, après la séparation du/des solvant(s), est séparé en une première fraction contenant du benzène, en une deuxième fraction contenant du toluène et en une troisième fraction contenant des xylènes.

6. Procédé selon l'une quelconque des revendications 1 à 5, **caractérisé en ce que** la distillation extractive est réalisée dans une première colonne de distillation, un flux hydrocarboné appauvri en aromatiques étant obtenu comme raffinat à bas point d'ébullition.

7. Procédé selon l'une quelconque des revendications 1 à 6, **caractérisé en ce que** la séparation du solvant est réalisée dans une deuxième colonne de distillation, un flux hydrocarboné enrichi en aromatiques étant obtenu comme raffinat à bas point d'ébullition.

8. Procédé selon l'une quelconque des revendications 1 à 7, **caractérisé en ce que** le solvant pour la distillation extractive est guidé dans un circuit fermé.

9. Procédé selon l'une quelconque des revendications 1 à 8, **caractérisé en ce que** le solvant d'extraction provenant de la séparation du/des solvant(s) peut être réinjecté comme produit de fond à point d'ébullition élevé depuis la deuxième colonne dans la partie supérieure de la première colonne.

10. Procédé selon l'une quelconque des revendications 1 à 9, **caractérisé en ce que** la perte de solvant peut être substituée par injection de solvant d'extraction ou d'un mélange de solvants d'extraction.

11. Procédé selon l'une quelconque des revendications 1 à 10, **caractérisé en ce que** la distillation extractive dans la première colonne est réalisée à une température de 200°C en l'extrémité inférieure de cette colonne et à une température de 50°C en l'extrémité supérieure de cette colonne.

12. Procédé selon l'une quelconque des revendications 1 à 11, **caractérisé en ce que** la séparation du/des solvant(s) dans la deuxième colonne est réalisée à une température de 260°C en l'extrémité inférieure de cette colonne et à une température de 50°C en l'extrémité supérieure de cette colonne.

13. Procédé selon l'une quelconque des revendications 1 à 12, **caractérisé en ce que** le flux de solvant(s) provenant de la séparation du/des solvant(s) est utilisé par des dispositifs d'échange thermique pour chauffer le mélange de départ pour la distillation extractive.

14. Procédé selon l'une quelconque des revendications 1 à 13, **caractérisé en ce que** le flux de solvant(s) provenant de la séparation du/des solvant(s) est utilisé par des dispositifs d'échange thermique pour chauffer la première colonne de distillation pour la distillation extractive.

15. Procédé selon l'une quelconque des revendications 1 à 14, **caractérisé en ce que** le flux de solvant(s) provenant de la séparation du/des solvant(s) est utilisé par des dispositifs d'échange thermique pour chauffer la deuxième colonne de distillation pour la distillation extractive.
